# EUROPÄISCHE PATENTANMELDUNG

(11) **EP 3 400 965 A1**
(43) Veröffentlichungstag der Anmeldung: **14.11.2018**
(21) Anmeldenummer: 18171966.7
(22) Anmeldetag: 13.05.2018
(51) Int. Cl.: A61K 47/46, A61K 9/70, A61K 36/185

(54) **ÖLIGE PFLANZENEXTRAKTE ENTHALTENDE ELEMENTE UND PFLASTER, DIE DERARTIGE WIRKSTOFFHALTIGE ELEMENTE ENTHALTEN**

(30) Priorität: 12.05.2017 DE 102017110405
(71) Anmelder: MSF Medizinische Sportwerke Frankfurt GmbH, 65929 Frankfurt (DE)
(72) Erfinder: HOFMANN, Wolfgang, 65929 Frankfurt (DE); MÜLLER, Walter, 65929 Frankfurt (DE); GRADER, Ludwig, 65929 Frankfurt (DE)
(74) Vertreter: Dr. Langfinger & Partner

(57) **Zusammenfassung**

Wirkstoffhaltiges filmförmiges Element, enthaltend
a) a) 25-85 Gew%, bezogen auf das Gesamtgewicht der Komponenten a) und b) im Endprodukt, einer vernetzbaren oder vernetzten Acrylatklebemasse oder Silikonharzklebemasse,
b) 15 bis 75 Gew%, bezogen auf das Gesamtgewicht der Komponenten a) und b) im Endprodukt, eines wirkstoffhaltigen öligen Pflanzenextrakts oder ätherischen Öles
mit einem Flächengewicht im Bereich von 5 bis 1000 g/m²

## Beschreibung

Die vorliegende Erfindung betrifft ölige Pflanzenextrakte enthaltende wirkstoffhaltige Elemente und Pflaster, die derartige Elemente enthalten.

Kosmetische Pflaster sind seit einer Reihe von Jahren bekannt.

Es sind zum einen in der Traditionswelt der Arzneimittel tief verwurzelte Produkte, zum anderen überwiegen hier, der Applikation des Zeitgeistes folgend, Produktanmutungen, die eher der Kosmetik zuzuordnen sind.

So spielen wärmende oder kühlende Produkte für Anwendungen in Sport, Kosmetik und Medizin seit Jahrtausenden eine bedeutende Rolle. Es gibt von vielen Herstellern wärmende bzw. je nach Anwendung kühlende Öle und Cremes zur Vorbereitung des Körpers auf den Sport, zur entspannenden Massage vor- und nach dem Sport und zur Steigerung der Beweglichkeit und allgemeinen Funktionsfähigkeit während des Sports. Dabei ist der Begriff "wärmend" oder "kühlend" neben der physikalischen Wärmebildung auch auf eine das Wärmegefühl oder Kältegefühl ansprechende stoffliche Wirkung hin erweitert zu sehen.

Zur Behandlung von muskulären, rheumatischen und Gelenkschmerzen gibt es Pflaster mit Capsaicinoiden als Wirkstoffe, welche dosisabhängig sehr starke Wärmereize setzen können. Die Wirkweise dieser Produkte beruht zum einen auf der Förderung der Durchblutung in den betroffenen Arealen und zum anderen auf einem Einfluss auf den Stoffwechsel oder auf die Weiterleitung des Schmerz-Reizes. Capsaicinoide reizen direkt die auf Wärmereize ansprechenden Schmerzrezeptoren der Haut und sind in geringer Konzentration bereits wirksam. Dies unterscheidet sie von vielen ausgesprochen mild wirksamen wärmenden (die Durchblutung fördernden) oder als kühlend bekannten Pflanzenextrakten z.B. in der Form ätherischer Öle.

WO2013/030227 beschreibt die Verwendung von ein Wärmegefühl erzeugenden, wirkstoffhaltigen Pflastern. Als Wirkstoffe werden durchblutungsfördernde Wirkstoffe (Vanillylbutylether, Carnitin, Capsaicin, Coffein) in einem Anteil von 0,01 bis 20 Gew. % in der Klebemasse unter Verwendung von vorzugsweise Decyloleat als Hilfsstoff beansprucht.

WO 2006/018340 beschreibt wirkstoffhaltige Tapes zur Behandlung von Gelenkerkrankungen.

DE-A 10056009 offenbart wirkstoffhaltige Matrixpflaster zur kontrollierten Abgabe von hyperämisierenden Wirkstoffen.

DE-A 19650471 beschreibt wirkstoffhaltige Pflaster mit einem Trägermaterial und einer darauf aufgebrachten Heißschmelzklebemasse, die mindestens einen hyperämisierenden Wirkstoff enthält. Die Klebmasse kann vollflächig oder partiell aufgetragen sein, letzteres um die Luft- und Wasserdampfdurchlässigkeit zu verbessern.

DE-A 19749467 beschreibt wirkstoffhaltige Pflaster mit einem Trägermaterial und einer darauf aufgebrachten Heißschmelzklebemasse, die mindestens einen Wirkstoff enthält, wobei die Klebmasse geschäumt ist.

In der EP 617972 wird ein schichtförmiges dermales therapeutisches System mit verzögerter Wirkstoffabgabe mit mindestens einem pharmazeutischem Wirkstoff beschrieben, welches eine oder mehrere Schichten aus Mischungen von Poly(meth)acyrylaten aufweist und welches aus einer Schmelze hergestellt wird. Des Weiteren wird ein Verfahren beschrieben, nach dem die Feststoffe der Polymerkomponente, des Wirkstoffs und ggf. weiterer Zusätze vermischt, die Mischung zur Schmelze erhitzt wird und das therapeutische System aus der geschmolzenen Masse durch deren Verteilung als dünne Schicht auf einem Träger hergestellt wird. Ölige Pflanzenextrakte werden in der Schrift nicht erwähnt. Der Gehalt an Wirkstoffen liegt in den Beispielen bei etwa 2 %.

Aus der EP 998 886 sind Heißkompressen bekannt, die als Wirkstoff bis zu 10 Gew% Ingwer-Extrakt enthalten.

Aus der WO 94/02123 sind Wirkstoffpflaster für niedrig schmelzende und/oder flüchtige Wirkstoffe bekannt. Diese enthalten einen Haftschmelzkleber auf Basis von elastomeren Styrolblockcopolymeren, d.h. bei Raumtemperatur ein elastisches Verhalten zeigenden Polymeren.

In der DE 196 50 471 werden wirkstoffhaltige Pflaster mit einem Trägermaterial und einer darauf aufgebrachten Heißschmelzselbstklebemasse beschrieben. Pflanzliche Extrakte werden nicht erwähnt.

In den vorstehend genannten Schriften wird die Verwendung von pflanzlichen Extrakten zwar teilweise erwähnt, doch liegen die Konzentrationen dieser Extrakte unter 10 Gew%, bezogen auf das Gesamtgewicht der Wirkstoffschicht im Endprodukt.

Wünschenswert ist es, kosmetische sowie auch therapeutische medizinische Pflaster in breiterem Umfang mit den Vorteilen einer wärmenden und/oder kühlenden Komponente zu verbinden. Um dies zu erreichen ist es weiterhin oftmals wünschenswert, natürliche Pflanzenextrakte zu verwenden, die aber aufgrund der Milde ihrer Wirkung in erheblichem Anteil an der Pflastermasse verwendet werden müssen, um überhaupt eine pflegende, erfrischende, andere kosmetische oder aber medizinische Wirkung zu entfalten. Die in diesen pflanzlichen Extrakten enthaltenen Bestandteile werden in verschiedenen Verfahren gewonnen und liegen aufgrund ihrer Fettlöslichkeit häufig als ölige Extrakte vor (z.B. ätherische Öle oder CO₂ Extrakte).

Ein Problem bei der Herstellung von Pflastern mit hohem öligem Gewichtsanteil in der Pflastermasse ist, dass die Pflastermasse durch das Öl sehr weich und wenig kohäsiv wird. Dies resultiert in einem Verhalten, das auch als "kalter Fluß" beschrieben wird. Er führt dazu, dass sich das Pflaster zunächst schlecht von der Abdeckfolie ("Release Liner") ablöst und/oder mit dem Packstoff verklebt. Es ist auch nach Gebrauch praktisch nicht möglich das Pflaster zu entfernen, ohne dass Reste auf der Haut zurück bleiben. Die zurückgebliebenen Reste müssen dann nach der Anwendung mühsam, oft mit organischen Lösemitteln wie z.B. Waschbenzin, entfernt werden. Dies schloss bisher die sinnvolle Verwendung ätherischer Öle, CO₂ Extrakte und anderer öliger Pflanzenextrakte in höherer Gewichtskonzentration praktisch aus.

Der vorliegenden Erfindung lag daher die Aufgabe zugrunde, wirkstoffhaltige Elemente, insbesondere in Pflastern, mit einem hohen Gehalt an ölhaltigem Extrakt zur Verfügung zu stellen, die die vorstehend geschilderten Nachteile nicht aufweisen. Diese Aufgabe wird erfindungsgemäß durch die wirkstoffhaltigen filmförmigen Elemente gemäß Anspruch 1 gelöst.

Bevorzugte Ausführungsformen der Erfindung sind den Unteransprüchen und der nachfolgenden detaillierten Beschreibung zu entnehmen.

Als "wirkstoffhaltiges Element" oder auch "wirkstoffhaltiges filmförmiges Element" wird im Folgenden jede einzelne wirkstoffhaltige Schicht eines Pflasters bezeichnet. Ein Pflaster kann ein einzelnes oder auch mehrere "wirkstoffhaltige (filmförmige) Elemente" und darüber hinaus auch weitere, in Pflastern übliche Komponenten enthalten.

Die wirkstoffhaltigen, filmförmigen Elemente gemäß der vorliegenden Erfindung enthalten
a) 25 bis 85 Gew%, vorzugsweise 35 bis 75 Gew% und besonders bevorzugt 40 bis 60 Gew%, bezogen auf das Gesamtgewicht der Komponenten a) und b) im Endprodukt, einer vernetzbaren oder vernetzten Acrylatklebemasse oder Silikonharzklebemasse, und
b) 15 bis 75 Gew%, vorzugsweise 25 bis 65 Gew%, besonders bevorzugt 40 bis 60 Gew.-%, bezogen auf das Gesamtgewicht der Komponenten a) und b) im Endprodukt, eines wirkstoffhaltigen öligen Pflanzenextrakts. und weisen ein Flächengewicht im Bereich von 5 bis 1000 g/m² auf.

Die vorstehenden Gewichtsangaben beziehen sich auf den Gehalt der Komponenten a) und b) im anwendungsfertigen Produkt, wie es vom Anwender als solches oder als Komponente eines Pflasters eingesetzt wird. Wie vorstehend erwähnt, kann ein fertiges Pflaster eines oder mehrere wirkstoffhaltige Elemente gemäß der Erfindung und darüber hinaus weitere Komponenten enthalten. Bei mehreren wirkstoffhaltigen Elementen beziehen sich die Prozentangaben auf jedes einzelne wirkstoffhaltige Element.

Die erfindungsgemäßen wirkstoffhaltigen Elemente werden durch Mischung der Komponenten a) und b) erhalten. Zum Zeitpunkt der Mischung kann sowohl die Komponente a) als auch die Komponente b) eines oder mehrere Lösungsmittel enthalten, die bei der Verarbeitung zum Endprodukt, wie es vom Anwender eingesetzt wird, mehr oder weniger vollständig entfernt werden. Insbesondere Acrylatklebemassen enthalten zum Zeitpunkt der Mischung mit der Komponente b) in der Regel Lösungsmittel, die nach der Mischung mit der Komponente b) beim anschließenden Vernetzen (die Mischung der Klebemasse mit der Komponente b) erfolgt in der Regel vor der Vernetzung der Klebemasse) weitgehend oder vollständig entfernt werden.

Auch ölige Pflanzenextrakte können neben dem Öl, welches die Grundlage des Extrakts bildet (der Wirkstoff selbst kann ölige Konsistenz haben) weitere Lösungsmittel zur Verbesserung der Handhabbarkeit und Verarbeitbarkeit enthalten.

Bei der Festlegung des Mischungsverhältnisses ist daher darauf zu achten, dass die Mengen der Komponenten a) und b) so gewählt werden, dass im anwendungsfertigen Endprodukt das Gewichtsverhältnis der Komponenten a) und b) im Bereich gemäß Anspruch 1 liegt.

Eine bevorzugte Gruppe von Acrylatklebemassen, die in den wirkstoffhaltigen Elementen gemäß der vorliegenden Erfindung eingesetzt werden können, sind (Meth)Acrylathomo- oder Copolymere, d.h. Polymere auf der Basis von Acrylsäure- oder Methacrylsäureestern, vorzugsweise den C₁-C₁₂-Alkylestern dieser Säuren.

Durch den Gehalt an Estern höherer Alkohole kann das plastische Verhalten (z.B. die Viskosität) der Polymeren eingestellt und an die Notwendigkeiten des konkreten Anwendungsfalls angepasst werden. Je höher der Anteil an höheren Alkylestern, desto weicher wird das Polymer. Dies geht mit einer Erniedrigung der Glasübergangstemperatur einher.

Bevorzugte Beispiele von (Meth)Acrylatcopolymeren sind Copolymere aus Methylmethacrylat und Ethylacrylat und Copolymere aus Methylmethacrylat und Butyl(meth)acrylat und/oder 2-Ethylhexylacrylat oder Copolymere aus Methylacrylat und Methylmethacrylat. Wie vorstehend erwähnt, kann das Schmelz- und Fließverhalten über das relative Verhältnis der Anteile an den jeweiligen Comonomeren gesteuert werden.

Neben den (Meth)Acrylatmonomeren können in den Acrylatklebemassen weitere Komponenten wie z.B. Vinylacetat oder Dicarbonsäureester mit enthalten sein.

Entsprechende Acrylatklebemassen sind dem Fachmann an sich bekannt und in der Literatur beschrieben, so dass sich nähere Angaben an dieser Stelle erübrigen.

Nur beispielhaft seien hier Klebemassen aus der Kombination der folgenden Monomeren genannt:
2-Ethylhexylacrylat/n-Butylacrylat/Butylmethacrylat/(Meth)Acrylsäure
2-Ethylhexylacrylat/n-Butylacrylat/Vinylacetat/(Meth)Acrylsäure
2-Ethylhexylacrylat/Vinylacetat/(Meth)Acrylsäure
2-Ethylhexylacrylat/Vinylacetat/Allyl(meth)acrylat
2-Ethylhexylacrylat/Vinylacetat/Divinylbenzol/(Meth)Acrylsäure;
2-Ethylhexylacrylat/Vinylacetat/Allylmethacrylat/(Meth)Acrylsäure
2-Ethylhexylacrylat/Vinylacetat/2-Hydroxyethyl(meth)acrylat
2-Ethylhexylacrylat/Fumarsäurediethylester oder
Maleinsäurediethylester/2-Hydroxyethyl(meth)acrylat.

Es hat sich herausgestellt, dass die Freigabe des Wirkstoffs aus dem wirkstoffhaltigen Element in einigen Fällen durch den Einbau funktioneller Gruppen in die Acrylatklebemasse beeinflusst und gesteuert werden kann.

Hier sind als bevorzugt Copolymerisate von Methacrylestern und Acrylestern mit funktionellen Gruppen zu nennen, wodurch die Polymere einen kationischen, anionischen oder hydrophilen Charakter erhalten. Comonomere, die solche funktionellen Gruppen in das Polymer einbringen, sind z-B. Acrylsäure, Methacrylsäure oder Maleinsäure für anonische Polymere oder Dimethylamino-ethylmethacrylat oder -acrylat, N-[3-(Dimethylamino)-2,2-dimethylpropyl]-methacrylamid für kationische Polymere und 2-Hydroxyethyl(meth)acrylat, 2-Hydroxypropyl(meth)acrylat und Trimethylammoniumethyl(meth)acrylatchlorid für Polymere mit hydrophilem Charakter.

(Methacrylat)polymere, die sich entsprechend als Komponente a) in den erfindungsgemäßen wirkstoffhaltigen Elementen eignen, sind in der Literatur umfassend beschrieben und von mehreren Herstellern z.B. unter den Handelsnamen Eudragit®, Plastoid® (beide von Evonik), Acryloid®, Paraloid® (Dow Chemical) erhältlich.

Geeignete Polymere mit funktionellen Gruppen sind z.B. in der EP-A 394956 (anionische (Meth)acrylatpolymere) und der EP-A 415055 (kationische Poly(meth)acrylate) beschrieben.

Geeignete Polymere a) sind auch in der EP-A 35399 beschreiben. Dabei handelt es sich um haftfähige Polyacrylate mit einem K-Wert im Bereich von 90 bis 110 (bestimmt nach DIN 53726), die aus 16-62 Gew% n-Butylacrylat, 34-80 Gew% 2-Ethylhexylacrylat und 4-10 Gew% Acrylsäure-Einheiten aufgebaut sind.

In manchen Anwendungsfällen haben sich Copolymere aus Methylmethacrylat und Butylmethacrylat als vorteilhaft erwiesen. Ein Beispiel solcher Polymere ist das von Dow Chemical unter der Bezeichnung Paraloid® B-66 erhältliche Produkt. Weitere geeignete Produkte sind unter dem Handelsnamen DuroTak® innerhalb des Geschäftsbereichs Loctite® von Fa. Henkel kommerziell erhältlich.

Hier seien nur beispielhaft folgende Duro Tak®-Produkte genannt: 87-2352, 387-2353, 87-235A, 387-235A, 87-2516, 387-2526, 87-2287, 387-2287, 87-2194, 387-2051, 387-2052, 387-2194, und 87-2196 genannt. Bei Duro Tak 87-2287 und DuroTak 387-2287 handelt es sich um Acrylat-Vinylacetat-Copolymere, die sich in einigen Fällen als besonders geeignet erwiesen haben..

GMS-9073, GMS-2873, GMS-9083, GMS-2883, GS-9067, GMS-9071, GMS-3083, GMS-3253, GMS-737, GMS-737-01, GMS 788, GMS-2999, GMS-2495, GMS-7883, GMS-1753, und GMS 2893 sind Beispiele für Acrlyatklebemassen, die von Fa. Cytec Corporation vertrieben werden und die erfindungsgemäß eingesetzt werden können.Weitere geeignete (Meth)acrylatpolymere, die unter dem Handelsnamen Paraloid erhältlich sind, sind Paraloid® B-44 (ein Copolymer aus Methylaycrylat und Methylmethacrylat)), Paraloid® B-67 (ein Isobutylmethacrylatpolymer) und Paraloid® B-72 (Copolymer aus Ethylacrylat und Methylmethacrylat) um nur einige weitere Vertreter zu nennen.

Die Acrylatklebemassen für die Herstellung von Pflastern etc. werden vorzugsweise in Form von Lösungen in organischen Lösungsmitteln verwendet. Dabei liegen sie in der Regel in unvernetzter Form vor, so dass ein geeigneter Vernetzer eingesetzt wird, um im Endprodukt die gewünschte Klebfähigkeit zu erreichen. Dabei bewirkt der Vernetzer die Verknüpfung der Polymerketten über reaktive Gruppen und erhöht so die Kohäsion der Klebemasse.

Bevorzugte Acrylatklebemassen in Form von organischen Lösungen sind die vorstehend bereits erwähnten Acrylatklebemassen auf der Basis der folgenden Monomeren
2-Ethylhexylacrylat/n-Butylacrylat/Butylmethacrylat/Acrylsäure
2-Ethylhexylacrylat/n-Butylacrylat/Vinylacetat/(Meth)Acrylsäure
2-Ethylhexylacrylat/Vinylacetat/(Meth)Acrylsäure
2-Ethylhexylacrylat/Vinylacetat/Allyl(meth)acrylat
2-Ethylhexylacrylat/Vinylacetat/Divinylbenzol/(Meth)Acrylsäure;
2-Ethylhexylacrylat/Vinylacetat/Allylmethacrylat/(Meth)Acrylsäure
2-Ethylhexylacrylat/Vinylacetat/2-Hydroxyethyl(meth)acrylat
2-Ethylhexylacrylat/Fumarsäurediethylester oder
Maleinsäurediethylester/2-Hydroxyethylacrylat.

Acrylatklebemassen können auch in Form wässriger Dispersionen eingesetzt werden. Diese haben ein Eigenschaftsspektrum, welches mit den Acrylatklebemassen in organischer Lösung vergleichbar ist, bieten jedoch den Vorteil, dass bei der Beschichtung und Trocknung keine brennbaren und giftigen Lösungsmittel anfallen.

Acrylatklebemassen in Form wäßriger Dispersionen, sog. Dispersionshaftkleber, haben sich in einigen Anwendungsfällen als vorteilhaft erwiesen und können vorteilhaft aus der Kombination folgender Monomerer polymerisiert werden:
n-Butylacrylat/Acrylsäure
2-Ethylhexylacrylat/n-Butylacrylat/Butylmethacrylat/(Meth)Acrylsäure
2-Ethylhexylacrylat/n-Butylacrylat/ (Meth)Acrylsäure
2-Ethylhexylacrylat/ n-Butylacrylat/2-Hydroxyethylacrylamid
2-Ethylhexylacrylat/ n-Butylacrylat /Vinylacetat/Acrylamid
2-Ethylhexylacrylat/ n-Butylacrylat/ Vinylacetat /2-Hydroxyethylacrylat;
2-Ethylhexylacrylat/n-Butylacrylatt/Allylacrylat/(Meth)Acrylsäure
2-Ethylhexylacrylat/n-Butylacrylat/Vinylacetat/ Divinylbenzol.

Anstatt einer Acrylatklebemasse können die erfindungsgemäßen wirkstoffhaltigen Elemente eine Silikonharzklebemasse enthalten. In einigen Fällen haben sich jedoch Acrylatklebemassen als vorteilhaft herausgestellt.

Silikonharzklebemassen sind dem Fachmann an sich bekannt und in der Literatur beschrieben, so dass sich nähere Angaben an dieser Stelle erübrigen.

Beispielhaft erwähnt seien hier Klebemassen auf der Basis von Siloxanen bzw. Polysiloxanen, wie z.B. Polydimethylsiloxan.

Die in den erfindungsgemäßen wirkstoffhaltigen Elementen eingesetzten Klebemassen sind vernetzbar oder vernetzt. Dies bedeutet, dass entweder bereits vernetzte Klebemassen zur Mischung mit der Komponente B eingesetzt werden oder aber dass die Klebemassen zum Zeitpunkt der Mischung einen noch aktiven Vernetzer enthalten. In der Regel erfolgt die Mischung der Komponente b) mit unvernetzter Klebemasse a).

Im letztgenannten Fall beträgt der Anteil dieses Vernetzers, bezogen auf das Gewicht von Klebemasse und Vernetzer bevorzugt 0,01 bis 10, besonders bevorzugt 0,02 bis 5 und insbesondere bevorzugt 0,02 bis 3 Gew%. Häufig sind Mengen an Vernetzer von 1 % oder weniger ausreichend. Die Prozentangaben beziehen sich in diesem Zusammenhang auf den Gehalt der Klebemasse an vernetzender Komponente zum Zeitpunkt der Mischung und nicht auf den Gehalt an Vernetzer im anwendungsfertigen wirkstoffhaltigen Element. Je nach Art des eingesetzten Vernetzers verbleiben nach der Aushärtung nur Teile des Vernetzermoleküls oder dessen Komponenten im Produkt.

Geeignete Vernetzer sind dem Fachmann bekannt und in der Literatur beschrieben.

Gemäß einer bevorzugten Ausführungsform ist bzw. sind als Vernetzer eine oder mehrere Verbindungen ausgewählt aus der Gruppe bestehend aus Diphenylmethandiisocyanaten, Hexamethylendiisocyanat, Isophorondiisocyanat, Acetylacetonaten, Alkyltitanaten, Alkyl-Alkohol-titanaten, polyfunktionellen Propyleniminderivaten, veretherten Melaminformaldehydharzen, Urethanharzen und Imino-Melaminharzen enthalten. Acetylacetonate werden im Allgemeinen bevorzugt.

Bevorzugte Acetylacetonate, die sich in einer Reihe von Anwendungen als vorteilhaft bewährt haben, sind Aluminiumacetylacetonat, Eisenacetylacetonat, Zinkacetylacetonat, Magnesiumacetylacetonat, Titanacetylacetonat und Zirkoniumacetylacetonat oder deren Mischungen.

In einigen Fällen hat es sich als vorteilhaft herausgestellt, wenn die in den erfindungsgemäßen wirkstoffhaltigen Elementen eingesetzten Acrylatklebemassen mindestens einen mit substituierter oder unsubstituierter (Meth)acylsäure veresterten Alkohol ausgewählt aus der Gruppe der langkettigen aliphatischen Alkohole, Fettalkohole, Wachsalkohole, Wollwachsalkohole und/oder Sterolen enthalten.

Durch die Mitverwendung solcher Komponenten kann die Benetzbarkeit der Hautoberfläche gesteuert werden. Entsprechende Komponenten sind aus der WO 2009/010120 bekannt und dort im Detail beschrieben, worauf hier wegen weiterer Einzelheiten verwiesen wird.

Unter dem Begriff Fettalkohole werden dabei die durch Reduktion der z.B. aus Triglyceriden oder Fettsäureestern, insbesondere Fettsäuremethylestern herstellbaren Fettsäuren erhältlichen linearen, gesättigten oder ungesättigten Alkohole mit vorzugsweise 6 bis 22 C-Atomen verstanden. Konkrete Beispiele sind der genannten WO 2009/010120 zu entnehmen.

Als Komponente b) enthalten die erfindungsgemäßen wirkstoffhaltigen Elemente 15 bis 75 , vorzugsweise 25 bis 65 und insbesondere 40 bis 60 Gew%, bezogen auf das Gesamtgewicht der Komponenten a) und b) im Endprodukt (wie vorstehend definiert), eines wirkstoffhaltigen öligen Pflanzenextrakts oder eines ätherischen Öls.

Unter dem Begriff "wirkstoffhaltiger öliger Pflanzenextrakt" sollen im Rahmen der vorliegenden Erfindung auch solche Produkte mit erfasst werden, die einen synthetisch hergestellten Wirkstoff in einem Trägeröl enthalten und deren Zusammensetzung einem Extrakt entspricht, wie er bei der Extraktion aus Pflanzen erhalten wird. Bevorzugt sind allerdings Extrakte mit Wirkstoffen, die durch Extraktion aus der Pflanze enthalten werden, die in der Regel auch unter wirtschaftlichen Gesichtspunkten vorteilhaft sind.

Nur beispielhaft seien hier einige Gruppen solcher wirkstoffhaltigen öligen Pflanzenextrakte, die in der Regel neben der Wirkstoffkomponente noch ätherische Öle enthalten, genannt:

Durchblutungsfördernde, Wärme vermittelnde ätherische Öle und pflanzliche Extrakte, insbesondere Ackerminze, Angelika, Angelikawurzel, Anis, Arnika, Baldrian, Basilikum, Bay, Beifuß, Benzoe, Bergamotte, Bohnenkraut, Cajeput, Cananga, Cassia, Davana, Douglasie, Edeltanne, Estragon, Eukalyptus, Fenchel, Fichte, Fichtennadel, Galbanum, Geranien, Gingergrass, Guajakholz, Ingwer, Johanniskraut, Kamille, Kampfer, Kardamom, Kiefer, Kiefernadelöl, Koriander, Kümmel, Latschenkiefer, Lavendel, Lemongrass, Liebstöckel, Limette, Lorbeer, Mairose, Majoran, Mandarine, Manuka, Meerkiefer, Melisse, Minze, Muskatellersalbei, Muskatnuss, Myrte, Nelke, Niaouli, Orange, Oregano, Origanum, Campbell Muse, Pfeffer, Pfefferminze, Rainfarn, Rosenholz, Rosmarin, römische Kamille, Salbei, Sandelholz, Sassafras, Schafgarbe, Schopflavendel, Sellerie, Tabak, Teebaum, Terpentin, Thuja, Thymian, Wacholder, Wacholderbeeren, Weißtanne, Wermut, Wiesenkönigin, Wintergrün, Ysop, Zeder, Zirkelkiefer, Zitrone, Zitronenmelisse und Zypresse, und

lokal das Gefühl von Kälte vermittelnde oder kühlende Substanzen, insbesondere Menthol, Minzöle, Wintergrünöl (Methylsalicylat), andere ätherische Öle und pflanzliche Extrakte, die unter anderem am Kälte-Menthol-Rezeptor (TRPM8) wirken oder auf sonstige Weise das Gefühl von Kälte vermitteln.

Gemäß einer bevorzugten Ausführungsform enthalten die erfindungsgemäßen wirkstoffhaltigen Elemente als öligen Pflanzenextrakt ein aus Pflanzen extrahiertes ätherisches Öl. Gemäß einer bevorzugten Ausführungsform handelt es sich bei den öligen Pflanzenextrakten um Oleoresine des Ingwer-Wurzelstocks. Der zähflüssige Balsam (Oleoresin) des Ingwer-Wurzelstocks ist auch beschrieben in der COSING Datenbank für kosmetische Inhaltsstoffe als Pflanzenauszug CAS 84696-15-1 von Zingiber Officinale. Dieser enthält die ätherischen Öle und einen Scharfstoffanteil an Gingerolen und Shogaolen, der vorzugsweise bei mindestens 10 Gew%, besonders bevorzugt bei mindestens 20 Gew%, bezogen auf das Gewicht des Extrakts liegt.

Neben den Komponenten a) und b) können die wirkstoffhaltigen Elemente gemäß der vorliegenden Erfindung weitere Komponenten enthalten, mit denen die Eigenschaften wie Viskosität oder andere anwendungstechnische Eigenschaften eingestellt werden können. Hier seien nur beispielhaft Füllstoffe, Hautdurchdringungsverstärker, Antioxidationsmittel und pflegende Bestandteile wie z.B. Öle (Yoyoba-Öl oder dgl.) oder Aloe-Vera Extrakte genannt. Diese können in einer Menge von 0-30 Gew%, bezogen auf das Gesamtgewicht im Endprodukt der Komponenten a) und b) in den erfindungsgemäßen wirkstoffhaltigen Elementen enthalten sein. Die Komponente b) reduziert sich dabei entsprechend. Pflegende Bestandteile wie Yoyoba oder Aloe Vera können aber auch in anderen Schichten enthalten sein.

Dem Fachmann sind entsprechende Zusätze bekannt und er wird auf der Grundlage seines Fachwissens im konkreten Anwendungsfall geeignete Zusätze auswählen, so dass sich hier detaillierte Angaben erübrigen.

Zur Herstellung der erfindungsgemäßen wirkstoffhaltigen Elemente werden die Komponenten a) und b) gemischt und die erhaltene Mischung zur Erzeugung eines filmförmigen Produkts mit einem Flächengewicht im Bereich von 5 bis 1000 g/m2 auf einen Träger aufgebracht. Das Flächengewicht des finalen filmförmigen Produkts liegt im Bereich von 5 bis 1000 g/m².

Als Trägermaterialien eignen sich alle starren und elastischen Flächengebilde aus synthetischen und natürlichen Rohstoffen. Bevorzugt sind Trägermaterialien, die nach Aufbringen der Mischung so eingesetzt werden können, dass sie Eigenschaften eines funktionsgerechten Verbandes erfüllen. Beispielhaft sind Textilien wie Gewebe, Gewirke, Gestricke, Gelege, Vliese, Laminate, Netze, Folien, Schäume oder Papiere zu nennen. Diese Materialien können einer Vor- oder Nachbehandlung unterworfen werden. Gängige Vorbehandlungen sind Corona- oder Plasmabehandlung und Hydrophobieren, gängige Nachbehandlungen sind Kalandern, Tempern, Kaschieren, Stanzen oder Eindecken.

In einigen Fällen hat es sich als vorteilhaft herausgestellt, wenn die Trägermaterialien aus einer luft- und wasserdampfdurchlässigen aber wasserundurchlässigen Polymerschicht bestehen. Hier seien als Beispiele Polymere ausgewählt aus Polyurethanen, Polyamiden, Polyethylen, Polypropylen, Polyestern, Polyetherestern oder auch thermoplastischen Elastomeren genannt, wie sie dem Fachmann bekannt und in der Literatur beschrieben sind. Soweit derartige Polymere in unmodifizierter Form keine ausreichende Wasserdampfdurchlässigkeit aufweisen, kann diese durch Verwendung entsprechend modifizierter Produkte, wie z.B. von Produkten mit mikroporöser Struktur, erhöht und angepasst werden.

In einigen Fällen haben sich textile Trägermaterialien auf der Basis von Polyamiden und/oder Elasthan als besonders geeignet herausgestellt.

Unter dem Begriff Elasthane werden dabei dehnbare synthetische Chemiefasern verstanden, die eine hohe Elastizität aufweisen. In der Regel sind diese aus Blockcopolymeren aufgebaut und weisen einen hohen Polyurethangehalt auf. Im englischsprachigen Raum sind entsprechende Produkte unter der Bezeichnung "spandex fibers" bekannt. Entsprechende Produkte sind dem Fachmann bekannt und in der Literatur beschrieben, so dass sich hier nähere Angaben erübrigen.

In einigen Fällen hat es sich als vorteilhaft herausgestellt, wenn zwei oder mehr wirkstoffhaltige Elemente als separate Lagen in definierter Reihenfolge mit unterschiedlichen pflanzlichen Komponenten auf das Trägermaterial aufgebracht werden, oder aber nur ein wirkstoffhaltiges Element verwendet wird, das mit einer zusätzlichen separaten Lage eines Klebstoffes mit oder ohne Zusatz pflegender Stoffe wie vorstehend beschrieben, wie z.B. Öle (Yoyoba-Öl oder dgl.) oder Aloe-Vera Extrakte, versehen wird. Durch die Verwendung zweier Lagen der Klebemassen kann eine Stabilisierung beim Auftragen auf die Haut erreicht werden. Zudem kann durch unterschiedliche Verteilung der Komponente b) und ggf. pflegender Bestandteile eine zeitlich modifizierbare Abgabe erreicht werden (z.B. zunächst verstärkte Freisetzung der Komponente b) und anschließend vermehrte Freigabe pflegender Bestandteile.

Die Mischung der Komponenten a) und b) kann durch Zugabe der Komponente b) zur Lösung oder Dispersion der Komponente a) erfolgen, wenn als Komponente a) die bevorzugten Acrylatklebemassen in Lösung oder Dispersion eingesetzt werden. Nach Zugabe der Komponente b) kann dann die Lösung oder Dispersion, die die Mischung aus a) und b) und ggf.de genannten weiteren Zusatzstoffe enthält, durch aufgießen oder aufsprühen auf den Träger aufgebracht und anschließend das Lösungsmittel z.B. durch Erhitzen entfernt werden. Die dafür anzuwendende Temperatur und der Zeitbedarf richten sich nach dem Siedepunkt des verwendeten Lösungsmittels.

Ein weiterer Gegenstand der vorliegenden Erfindung ist ein Pflaster, welches ein wirkstoffhaltiges Element gemäß der Erfindung enthält.

Der hier verwendete Begriff des Pflasters umfasst neben klebenden kosmetisch/medizinischen Pflastern oder Matrices alle kosmetisch/medizinisch anwendbaren Hautauflagen wie Patch, Pad, Tapes, Tücher, Binden, Dressings, Cataplasmen, Bandagen und/oder Masken. Selbstklebend bedeutet hierbei, dass die Hautauflage aufgrund der Klebemasse auf der Haut haftet und nicht durch zusätzliche Befestigungsvorrichtungen oder Massen fixiert werden müssen. Pflaster mit Acrylatklebemassen zeichnen sich in der Regel durch ein sehr geringes allergenes Potential aus.

Pflaster enthalten neben den vorstehend beschriebenen Komponenten in der Regel eine vor Gebrauch zu entfernende Abdeckfolie, den sogenannten Release-Liner.

Als Release-Liner werden in der Regel nicht haftende Trägerfolien, von denen das wirkstoffhaltige Element bzw. Pflaster leicht wieder abgezogen werden kann, eingesetzt. Entsprechende Produkte sind dem mit der Herstellung von Pflastern vertrauten Fachmann bekannt und in der Literatur beschrieben, so dass sich hier nähere Angaben erübrigen. Der Fachmann wird unter Zuhilfenahme seiner Erfahrung das für den konkreten Anwendungsfall am besten geeignete Material für den Release-Liner auswählen. Beispiele für geeignete Materialien sind Polyethylen, Polypropylen sowie Polyethylenterephthalat. Weiterhin wären zu nennen silikonisierte oder anderweitig antihaftbeschichtete Folien der vorstehend beschrieben Art oder Silikonpapiere.

Nach einem Verfahren zur Herstellung der wirkstoffhaltigen Elemente gemäß der vorliegenden Erfindung werden in einem ersten Schritt Klebemasse und öliger Pflanzenextrakt in einem solchen Verhältnis gemischt, dass das erhaltene Produkt 25 bis 85 Gew%, bezogen auf das Gesamtgewicht beider Komponenten a) und b) im Endprodukt, an Acrylatklebemasse und 15 bis 75 Gew% an öligem Extrakt enthält. Anschließend wird die Mischung auf einen Träger aufgebracht, wozu vorzugsweise eine Düse eingesetzt werden kann, wenn die Mischung in Lösung oder Dispersion vorliegt, was sich in einigen Fällen als vorteilhaft erwiesen hat. Es ist jedoch genauso möglich, die Mischung aus a) und b) in Form einer Dispersion oder Lösung durch Aufgießen auf den Träger aufzubringen und anschließend das Lösungsmittel zu entfernen. Auch eine Beschichtung mit Rakel oder Rollcoater ist möglich.

Prinzipiell ist es auch möglich, eine schmelzflüssige Mischung aus a) und b) auf einen Träger aufzutragen (sog. Schmelzhaftkleber); dies wird jedoch in der Regel nicht bevorzugt.

Falls ölige Pflanzenextrakte eingesetzt werden, die flüchtig sind und daher beim Erhitzen möglicherweise verdampfen oder abgebaut werden, kann man das Entfernen des Lösungsmittels unter verringertem Druck ausführen, wodurch es möglich wird, die erforderliche Temperatur zu verringern.

Die Dicke der erfindungsgemäßen wirkstoffhaltigen Elemente unterliegt keiner besonderen Beschränkung.

Das Flächengewicht des nach Entfernen des Lösungsmittels erhaltenen wirkstoffhaltigen Elements liegt im Bereich von 5 bis 1000 g/m², vorzugsweise im Bereich von 10 bis 500 g/m².

Das so erhaltene wirkstoffhaltige Element gemäß der vorliegenden Erfindung kann entweder im gleichen Arbeitsgang oder zu einem späteren Zeitpunkt zu einem kosmetischen oder medizinischen Pflaster weiter verarbeitet werden. Hierzu kann z.B. der dünne erhaltene Film durch entsprechende Laminierungsschritte auf beiden Seiten entsprechend [0062] und [0069] mit einem weiteren Pflaster-Klebstoff (typischerweise einem Acrylatkleber) beschichtet und der resultierende Aufbau mit einer geeigneten Rückschicht (Gewebe, Filz; Vlies, Gestrick oder Gewirk etc.) versehen werden.

Alternativ kann das erfindungsgemäße wirkstoffhaltige Element ohne besondere eigene klebende Beschichtung auch als Wickel oder Umschlag verwendet werden (z.B. für Heilungsprozesse als Umschlag).

Zwischen Pflaster-Rückenmaterial (der der Haut abgewandten Oberfläche des Pflasters) und dem wirkstoffhaltigen Element kann eine Schutzschicht (z.B. Polyisobutylen, PIB) aufgetragen werden, um die Diffusion und Aufnahme des Wirkstoffes durch das Pflaster-Rückenmaterial zu verhindern oder zumindest zu verringern und damit einem Verlust der Wirksubstanz über das Pflaster-Rückenmaterial vorzubeugen.

Gemäß einer weiteren Ausführungsform der Erfindung kann das wirkstoffhaltige Element durch gezielte Einbringung von Lücken oder Dicke- Unterschieden gestaltet werden, um bestimmte physikalische / Flexibilitäts-Eigenschaften des Gesamtverbandes zu erhalten bzw. zu verstärken (z.B. Anwendung in kinesiologischen Tapes).

Das Gemisch aus Klebemasse und öligem Pflanzenextrakt kann gezielt mit speziellen Beschichtungsverfahren zu geometrisch vorgegebenen einfachen oder komplexen Mustern auf einen Release Liner (zur weiteren Verwendung im Rahmen der Pflaster - Schichtenherstellung) oder direkt auf eine geeignete vorliegende Schicht eines Pflasters aufgetragen werden, um räumlich differenzierte Reiz-Effekte zu erzielen.

Zur Verbesserung der Handhabbarkeit kann eine zweite Schutzfolie in Form einer nicht haftenden Trägerfolie (Release-Liner) auf die noch offene Oberfläche des erhaltenen wirkstoffhaltigen Elements aufgebracht werden.

Die vorliegende Erfindung ermöglicht die Verwendung großer Volumenanteile flüssiger oder öliger Substanzen, wie z.B. pflanzlicher CO₂ Extrakte. Diese führen normalerweise zu einer Verflüssigung des klebenden Pflastermateriales, was zur Unbrauchbarkeit des Pflasters führt.

Eine Eigenschaft vieler wärmender oder kühlender pflanzlicher Extrakte, so auch der Zingiber Officinale Oleoresin Extrakte ist, dass sie im Vergleich z.B. zu den arzneiliche Verwendung findenden Capsaicinoiden deutlich schwächer durchblutungsfördernd sind. Sie müssen daher, um überhaupt eine wahrnehmbar angenehme pflegende und wohltuende Wirkung auf der Haut zu entfalten in höherer Konzentration eingesetzt werden als die Capsaicinoide. In der Verwendung in selbstklebenden Hautauflagen ist der resultierende hohe Oleoresin Gehalt aber ein technisches Problem, als dass er ohne weitere Vorkehrungen die Klebkraft der Gesamtmatrix reduziert sowie die Polymerisierung der unter dem Gewebe liegenden Gesamtschicht so beeinflusst, dass Konsistenz und Stabilität der Gesamtstruktur beeinträchtigt ist. Das Resultat ist zumeist eine Auflösung der extrakthaltigen Matrix während des Tragens, verbunden mit einem Zurückbleiben von unangenehmen Kleber Resten auf der Haut nach Entfernung des Pflasters. Diese Probleme werden mit der vorliegenden Erfindung vermieden oder zumindest deutlich reduziert.

Die vorliegende Erfindung beschreibt somit erstmals eine technische Lösung, die die Verwendung von öligen pflanzlichen Extrakten, wie zum Beispiel Zingiber Officinale Oleoresin Extrakten (z.B. CO₂ Extrakten) in höherer Konzentration in kosmetischen und medizinischen selbstklebenden Hautauflagen ermöglicht, ohne die beschriebenen unerwünschten Wirkungen der Auflösung des Gesamtverbandes zu zeigen.

Beispiel: Pflasteraufbau mit einem textilen Träger und zwei Klebstoffschichten
a. ein hautseitig angebrachtes filmförmiges wirkstoffhaltiges Element gemäß der Erfindung enthaltend:
   i. Lösemittelbasierten Acrylatklebstoff auf Basis n-ButylAcrylat/Vinylacetat mit 51% Feststoffgehalt und einer Viskosität von 18000 mPas (DuroTak® 87-2287, erhalten von Henkel): 51,23 Gew%
   ii. Ingwer Oleoresin CO₂ Extrakt mit 30% Gingerolen und Shogaolen: 46,25 Gew%
   iii. Titan Acetyl Acetonat: 2,52 Gew%
   iv. mit einem Auftragsgewicht von 30g/m²
b. eine weitere Schicht enthaltend
   i. Lösemittelbasierten Acrylatklebstoff wie in a): 79,99%
   ii. Yoyoba Öl 18,32 Gew%
   iii. Aloe Vera Pulver 1,69 Gew%
   iv. mit einem Auftragsgewicht von 100g/m²
c. einem Gewebe bestehend aus Polyester und Elastan, das als Trägermaterial dient
   Zum Schutz des Pflasteraufbaus wird ein Release Liner auf die Schicht a) aufgebracht, der vor Anwendung abgezogen wird. Der so hergestellte Aufbau besitzt die positiven elastischen Eigenschaften des Trägermaterials und der Acrylatkleber Schichten, vermittelt zunächst merkbar aber pfleglich ein tiefes, lang anhaltendes sehr wohltuendes Wärmegefühl an der Haut welches entspannt und die Beweglichkeit fördert, und pflegt anschließend die Haut weiter durch die zusätzlichen pflegenden Komponenten.

## Patentansprüche

1. Wirkstoffhaltiges filmförmiges Element, enthaltend
a. 25 bis 85 Gew%, bezogen auf das Gesamtgewicht der Komponenten a) und b) im Endprodukt, einer vernetzbaren oder vernetzten Acrylatklebemasse oder Silikonharzklebemasse,
b. 15 bis 75 Gew%, bezogen auf das Gesamtgewicht der Komponenten a) und b) im Endprodukt, eines wirkstoffhaltigen öligen Pflanzenextrakts oder eines ätherischen Öles
c. mit einem Flächengewicht im Bereich von 5 bis 1000 g/m².

2. Wirkstoffhaltiges Element nach Anspruch 1, **gekennzeichnet durch** einen Gehalt der Komponente b) im Bereich von 25 bis 65, vorzugsweise 40 bis 60 Gew%, bezogen auf das Gesamtgewicht der Komponenten a) und b) im Endprodukt.

3. Wirkstoffhaltiges Element nach einem der Ansprüche 1 oder 2, enthaltend als öligen Pflanzenextrakt ein aus Pflanzen extrahiertes ätherisches Öl.

4. Wirkstoffhaltiges Element nach einem der Ansprüche 1 oder 2, **dadurch gekennzeichnet, dass** der ölige Pflanzenextrakt b) Scharfstoffe des Ingwer enthält.

5. Wirkstoffhaltiges Element nach Anspruch 3, **dadurch gekennzeichnet, dass** der ölige Pflanzenextrakt mindestens 10 Gew%, bezogen auf Gewicht des Pflanzenenxtrakts, an Gingerolen und/oder Shogaolen enthält.

6. Wirkstoffhaltiges Element nach einem der Ansprüche 1 bis 5, **dadurch gekennzeichnet, dass** die Acrylat- oder Silikonharzklebemasse vor der Vernetzung einen Vernetzer in einer Menge von 0,01 bis 10 Gew. %, bezogen auf das Gesamtgewicht der Klebemasse enthält.

7. Wirkstoffhaltiges Element nach Anspruch 6, **dadurch gekennzeichnet, dass** als Vernetzer eine oder mehrere Verbindungen ausgewählt aus der Gruppe bestehend aus Diphenylmethandiisocyanaten, Hexamethylendiisocyanat, Isophorondiisocyanat, Acetylacetonaten, Alkyltitanaten, Alkyl-Alkohol-titanaten, polyfunktionellen Propyleniminderivaten, veretherten Melaminformaldehydharzen, Urethanharzen und Imino-Melaminharzen enthalten ist.

8. Wirkstoffhaltiges Element nach Anspruch 7, **dadurch gekennzeichnet, dass** das Acetylacetonat ausgewählt ist aus Aluminiumacetylacetonat, Eisenacetylacetonat, Zinkacetylacetonat, Magnesiumacetylacetonat, Titanacetylacetonat und Zirkoniumacetylacetonat.

9. Selbstklebendes Pflaster, enthaltend mindestens ein wirkstoffhaltiges Element nach einem der Ansprüche 1 bis 8.

10. Selbstklebendes Pflaster nach Anspruch 9, enthaltend neben dem wirkstoffhaltigen Element eine Trägerschicht und eine Schutzfolie.

11. Verwendung eines wirkstoffhaltigen Elements nach einem der Ansprüche 1 bis 8 zur Herstellung eines wirkstoffhaltigen Pflasters.
